Europäisches Patentamt

European Patent Office

Office européen des brevets

(19)

(11) Veröffentlichungsnummer: **0 143 453**
A2

(12) # EUROPÄISCHE PATENTANMELDUNG

(21) Anmeldenummer: **84114233.4**

(22) Anmeldetag: **24.11.84**

(51) Int. Cl.⁴: **A 61 N 1/42**

(30) Priorität: **28.11.83 DE 3342987**

(43) Veröffentlichungstag der Anmeldung: **05.06.85**
Patentblatt 85/23

(84) Benannte Vertragsstaaten: **AT CH FR GB IT LI NL**

(71) Anmelder: **Schauf, G., Prof., Parkstrasse 26,
D-5600 Wuppertal 21 (DE)**

(72) Erfinder: **Schauf, G., Prof., Parkstrasse 26,
D-5600 Wuppertal 21 (DE)**

(74) Vertreter: **Patentanwälte Dr. Solf & Zapf,
Schlossbleiche 20 Postfach 13 01 13,
D-5600 Wuppertal 1 (DE)**

(54) **Magnetfeld-Therapiegerät mit Behandlungssonden.**

(57) Zunehmend werden niederfrequente Magnetfelder in der medizinischen Therapie eingesetzt. Dabei verwendet man vorrangig sogenannte Luftspulen, also Spulen bei denen innerhalb der Spule kein ferromagnetisches Material eingesetzt wird. Mit den Luftspulen ist nur eine grossräumige Behandlung möglich.

Das hier als neu vorgeschlagene System erlaubt eine gezielte punktuelle Behandlung mit definierten Feldern und Feldänderungen. Das Gerät soll mit einstellbaren Magnetpolen das Feld konzentriert und ohne wesentliches Streufeld an den jeweiligen Behandlungsort leiten. Das Magnetfeld wird in einer langen Spule (1) erzeugt und durch einen ferromagnetischen Leiter (3) zum Behandlungsort (7) übertragen. Die Behandlungsfläche wird durch die auswechselbaren Polschuhe (6) bestimmt.

Prof G SCHAUF
Wuppertal, Parkstr 26

0143453

- 1 -

Magnetfeldtherapiegerät mit **Behandlungssonden**

_____

Beschreibung
--------------

Stand der Technik:

Zunehmend werden niederfrequente Magnetfelder in der medizinischen Therapie eingesetzt. Über die Wirkungsweise besteht noch große Unsicherheit, ebenfalls über Anwendungsbereiche und Risiken, obwohl man bisher davon ausgeht, daß Magnetfelder unschädlich für den menschlichen Organismus sind.

Zum Einsatz kommen bei allen bekannten Behandlungssystemen sogenannte Luftspulen, Spulen bei denen also innerhalb der Spule kein ferromagnetisches Material eingesetzt wird. In die Spule wird vielmehr der zu behandelnde Körper oder Köperteil eingebracht und so dem Magnetfeld ausgesetzt. In eingen Fällen wird die Spule auch als Primärwicklung zu einem implantierten sekundären Empfangsteil (Übertrager) benutzt, um eine sekundäre elektrische Reizung von Körpersubstanz zu erreichen.

Untersuchungen verschiedener Stellen haben gezeigt, daß mit bestimmten Einwirkungen Kalzifizierungen erreicht werden, was sowohl positiv als auch negativ sein kann.

Mangel bisheriger Lösungen:

Bisherige Behandlungsgeräte setzen Magnetspulen mit großem Streufeld ein. Der Patient wird in die Spule eingebracht, die Spule ist also in der Regel groß und wirkt auf den gesamten Umraum (Patient, Behandlungspersonen und benachbarte Geräte). Da die Geräte teilweise sogar mit Rechtecksignalen betrieben werden, ist das abgestrahlte Frequenzspektrum auch hochfrequent und breitbandig.
Der therapeutische Mangel ist sicher, daß keine gezielt lokale Behandlung möglich ist - der technische die Störmöglichkeit für Nachbargeräte.

Zweck der Erfindung:

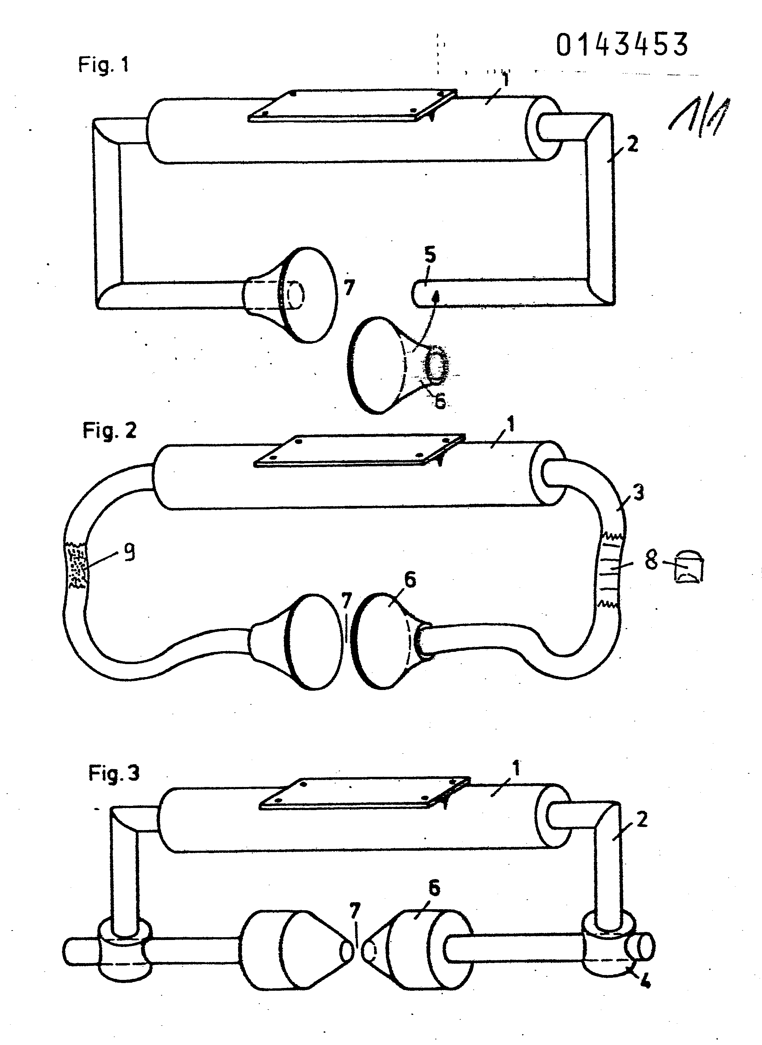

Die nachfolgend beschriebene Lösung soll es ermöglichen, ein möglichst räumlich definiertes Feld, homogen oder inhomogen (Gradientenfeld), zu erzeugen, das in einem begrenzten Raumteil gezielte Therapie erlaubt. Die Lösung soll mit einstellbaren Magnetpolen das Feld konzentriert und ohne wesentliches Streufeld an den jeweiligen Behandlungsort leiten. Durch die Auswertung der Strom- und Spannungswerte ist bei Eingabe der Polparameter eine Mikroprozessor-Feldsteuerung/Feldregelung möglich. Der Einsatz von flexiblen ferromagnetischen Übertragern - ferromagnetisches Kabel -, gefüllt mit ferromagnetischem Staub oder Granulat, oder der Einsatz von geketteten ferromagnetischen Stäben mit kugelförmiger Anpassung (Figur 2. Element 8), läßt es zu, daß die Polschuhe verschiedener Form als Magnetpole am Behandlungsvolumen wirksam werden.

Lösung der Aufgabenstellung:

Die Aufgabe wird dadurch gelöst, daß das Magnetfeld in einer langen Spule (1) erzeugt und durch einen ferromagnetischen Uebertrager (2), der starr (2) oder flexibel (3), verstellbar (4) oder fest eingestellt sein kann, zu den ferromagnetischen Enden (5), die mit einem Polschuh (6) bestücktsein können, geleitet wird. Die Abstände der Pole zu einander sind dabei deutlich kleiner als die Spulenlänge. Somit ist das Streufeld klein gegenüber dem Behandlungsraum (7).

Die Pole können am Körper direkt angebracht sein, durch ein Stativ gehalten werden oder mit der Spule fest verbunden sein.
Durch Schrägstellung oder Konturierung der Pole kann eine zusätzliche Beeinflussung des Feldes - besonders der gewollten Inhomogenität - erreicht werden, ebenso eine Homogenisierung.

Die Feldabhängigkeit vom Polabstand wird durch automatische Strom- und Spannungsauswertung der Spule, bei eingegebener Polschuhgröße, ausgeregelt über ein digitales Rechenprogramm (Mikroprozessor). Es besteht alternativ die Möglichkeit der Feldregelung auf der Basis einer Feldmessung unter dem Polschuh (Hallsondeneinbau).

Erzielbarer Vorteil:

Die Spulen- und Magnetpolanordnung erlaubt die gezielte Behandlung eines zu therapierenden Bereichs, ohne daß durch ein wesentliches Streufeld eine Beeinflussung des Nachbarbereiches in Kauf genommen werden muß. Mit Hilfe fixierter Pole ist die gezielte Behandlung sowohl mit homogenen als auch inhomogenen Feldern möglich.

Durch die direkte Feldmessung unter dem Behandlungspol, alternativ die indirekte Ermittlung des Feldes aus Poltyp, Strom und Spannung, ist eine automatische Feldeinstellung auf den Sollwert gegeben.

Patentansprüche
----------------------------

1. Magnetfeldtherapiegerät bestehend aus der Ansteuerelektronik, einer Spule (1), die das Magnetfeld erzeugt, und dem Uebertragungssystem (2), d a d u r c h   g e k e n n z e i c h n e t, daß das von einer langen Spule erzeugte Magnetfeld ferromagnetisch über starre (2) und/oder flexible (3) und/oder verstellbare (4) Verbindung zu den Behandlungsenden unterschiedlicher Gestaltung überträgt, wobei die Spule deutlich länger ist als der Abstand zwischen den Behandlungsköpfen (7) - in der der Regel mindestens doppelt so lang, um das Streufeld zu beschränken.

2. Gerät nach 1, d a d u r c h   g e k e n n z e i c h n e t, daß der ferromagnetische Uebertrager entweder aus Ferrit-Teilkörpern besteht (8), die durch Kugelgelenk miteinander verbunden sind, oder der auch ganz oder teilweise aus anderem ferromagnetischem Material bestehen kann - auch Ferritstauben oder Granulaten (9).

3. Gerät nach 1, d a d u r c h   g e k e n n z e i c h n e t, daß an den Enden der Uebertrager Polschuhe (6) befestigt werden können, die den Behandlungsraum bestimmen.

4. Gerät nach 1, d a d u r c h   g e k e n n z e i c h n e t, daß die Polschuhe (6) zur Anpassung an den Körper plastisch ausgeführt sein können - z.B. Beutel mit ferromagnetischen Partikeln.

5. Gerät nach 1, d a d u r c h   g e k e n n z e i c h n e t, daß unter den Polen Magnetfeldsonden untergebracht sein können, die zur Messung des tatsächlichen Behandlungsfeldes genutzt werden.

6. Gerät nach 1, d a d u r c h   g e k e n n z e i c h n e t, daß die Ansteuerelektronik auf der Basis von 5 oder der Auswertung von Strom und Spannungswerten der Spule eine automatische Feldeinstellung nach Intensität und zeitlichem Verlauf vornehmen kann.

0143453

Fig. 1

Fig. 2

Fig. 3